(19) Europäisches Patentamt

European Patent Office

Office européen des brevets

(11) **EP 4 404 207 A1**

(12) **EUROPEAN PATENT APPLICATION**

(43) Date of publication:
**24.07.2024 Bulletin 2024/30**

(21) Application number: **24152572.4**

(22) Date of filing: **18.01.2024**

(51) International Patent Classification (IPC):
**G16H 30/40** (2018.01) **G06N 3/045** (2023.01)
**G16H 50/20** (2018.01) **G16H 50/70** (2018.01)

(52) Cooperative Patent Classification (CPC):
**G16H 30/40; G06N 3/045; G16H 50/20;
G16H 50/70**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(30) Priority: **18.01.2023 US 202318155781**

(71) Applicant: **Siemens Healthineers AG
91301 Forchheim (DE)**

(72) Inventor: **GRBIC, Sasa
Plainsboro, NJ, 08536 (US)**

(74) Representative: **Horn Kleimann Waitzhofer
Schmid-Dreyer
Patent- und Rechtsanwälte PartG mbB
Theresienhöhe 12
80339 München (DE)**

(54) **AUTOMATIC PERSONALIZATION OF AI SYSTEMS FOR MEDICAL IMAGING ANALYSIS**

(57)    Systems and methods for personalizing an AI (artificial intelligence) system for medical imaging analysis are provided. One or more input medical images are received. A medical imaging analysis task is performed on the one or more input medical images using a trained AI system. Feedback on results of the medical imaging analysis task is received from a user. The trained AI system is retrained based on the feedback to generate a user-specific AI system for the user. The user-specific AI system is validated. The validated user-specific AI system is output.

FIG. 2

EP 4 404 207 A1

**Description**

TECHNICAL FIELD

The present invention relates generally to AI (artificial intelligence) systems for medical imaging analysis, and in particular to automatic personalization of AI systems for medical imaging analysis.

BACKGROUND

Recently, AI (artificial intelligence) systems have been developed for performing various medical imaging analysis tasks, such as, e.g., detection, classification, segmentation, quantification, etc. Such AI systems are often trained with supervised learning using annotated training data. The training data is typically annotated by experts with different preferences and understandings of what is being annotated. The different preferences and understandings may be due to regional differences that may reflect the local patient population and disease prevalence. Accordingly, users are often unsatisfied with AI systems trained with such annotated training data.

Currently, AI systems generally do not have the ability to be personalized to a user's preferences. For example, an AI system for the detection of lung nodules will be the same no matter where the AI system is sold and used. However, as users have different preferences, this will create challenges for user acceptance of results of the AI system.

BRIEF SUMMARY OF THE INVENTION

The invention is defined by the enclosed claims, wherein in accordance with one or more embodiments, systems and methods for personalizing an AI (artificial intelligence) system for medical imaging analysis are provided. One or more input medical images are received. A medical imaging analysis task is performed on the one or more input medical images using a trained AI system. Feedback on results of the medical imaging analysis task is received from a user. The trained AI system is retrained based on the feedback to generate a user-specific AI system for the user. The user-specific AI system is validated. The validated user-specific AI system is output.

In one embodiment, the user-specific AI system is validated by performing a medical imaging analysis validation task on a validation dataset using the user-specific AI system. A performance of the user-specific AI system is determined by comparing results of the medical imaging analysis validation task with ground truth annotations of the validation dataset. The performance of the user-specific AI system is compared with a performance threshold.

In one embodiment, the feedback comprises one or more of acceptance of the results, rejection of the results, editing of the results, or creation of new results. The feedback is stored on a database associated with the user.

In one embodiment, the trained AI system is retrained based on the feedback from only the user.

In one embodiment, the validated user-specific AI system is deployed for use in a clinical setting.

In one embodiment, the medical imaging analysis task comprises detection of abnormalities.

These and other advantages of the invention will be apparent to those of ordinary skill in the art by reference to the following detailed description and the accompanying drawings.

BRIEF DESCRIPTION OF THE DRAWINGS

Figure 1 shows a method for personalizing an AI system, in accordance with one or more embodiments;

Figure 2 shows a workflow for personalizing an AI system, in accordance with one or more embodiments;

Figure 3 shows an exemplary artificial neural network that may be used to implement one or more embodiments;

Figure 4 shows a convolutional neural network that may be used to implement one or more embodiments; and

Figure 5 shows a high-level block diagram of a computer that may be used to implement one or more embodiments.

DETAILED DESCRIPTION

The present invention generally relates to methods and systems for automatic personalization of AI (artificial intelligence) systems for medical imaging analysis. Embodiments of the present invention are described herein to give a visual understanding of such methods and systems. A digital image is often composed of digital representations of one or more objects (or shapes). The digital representation of an object is often described herein in terms of identifying and manipulating the objects. Such manipulations are virtual manipulations accomplished in the memory or other circuitry/hardware of a computer system. Accordingly, is to be understood that embodiments of the present invention may be performed within a computer system using data stored within the computer system. It is understood that when it is referred to a "means or device for" executing an action the "means or device is configured to or implemented to" carry out the action. In particular, the means/device may be a structural embodiment of a method step or process including the action, e. g. a "means for receiving" may corespond to the "step of receiving".

Embodiments described herein provide for the automatic personalization of AI (or machine learning) systems to generate user-specific AI systems for medical imaging analysis. In particular, embodiments described herein provide for a modular AI system that tracks user feedback and personalizes itself based on the user feedback on a regular schedule. The personalization of the AI system is performed by retraining the AI system with the user feedback to generate a user-specific AI system. In order to ensure the AI system is not experiencing regression, the retrained user-specific AI system is validated before deploying the retrained user-specific AI system. Advantageously, embodiments described herein provides improved acceptance of AI results of users as the AI system is personalized based on the user feedback, provides for improved efficiency in the radiologist reading workflow as the AI system and the user improve their collaborative work over time due to the AI system becoming more and more personalized as the user provides more and more feedback, and provides for increased utilization of the AI system due to improve user acceptance of results.

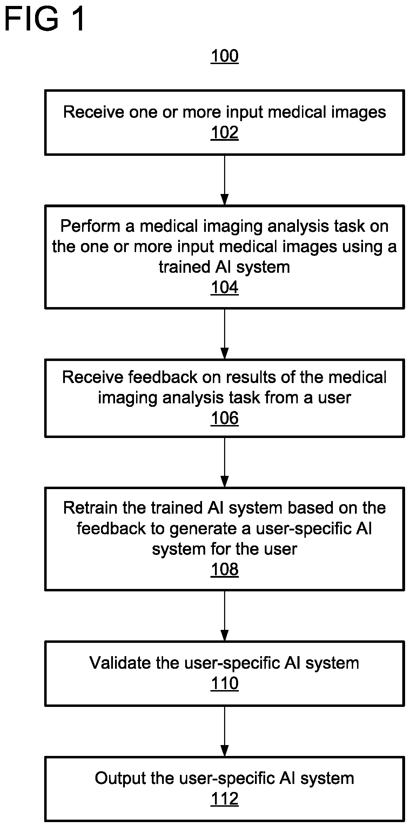

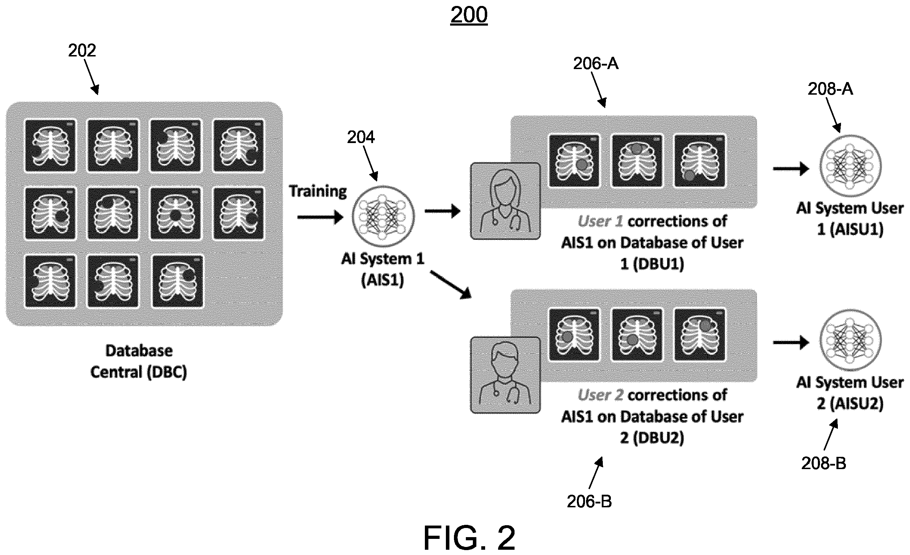

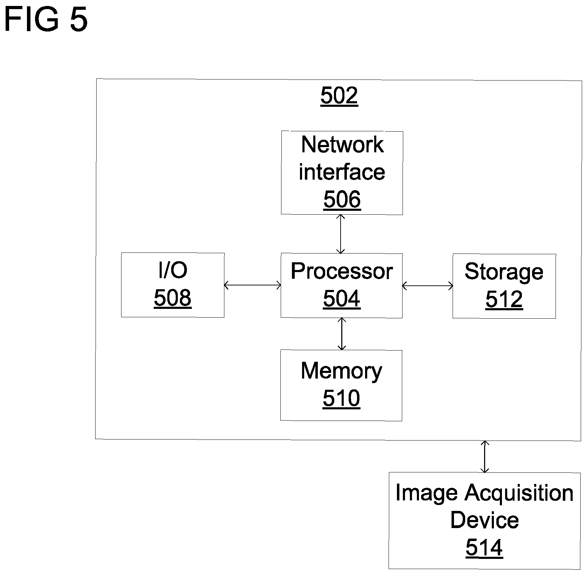

Figure 1 shows a method 100 for personalizing an AI system, in accordance with one or more embodiments. The steps of method 100 may be performed by one or more suitable computing devices, such as, e.g., computer 502 of Figure 5. Figure 2 shows a workflow 200 for personalizing an AI system, in accordance with one or more embodiments. Figure 1 and Figure 2 will be described together.

At step 102 of Figure 1, one or more input medical images are received. The one or more input medical images may depict any anatomical object of interest, such as, e.g., organs, vessels, bones, lesions, etc. The one or more input medical images may comprise any suitable modality, such as, e.g., CT (computed tomography), MRI (magnetic resonance imaging), ultrasound, x-ray, or any other medical imaging modality or combinations of medical imaging modalities. The one or more input medical images may be 2D (two dimensional) images and/or 3D (three dimensional) volumes, and may comprise a single input medical image or a plurality of input medical images. The one or more input medical images may be received directly from an image acquisition device, such as, e.g., a CT scanner, as the medical images are acquired, or can be received by loading previously acquired medical images from a storage or memory of a computer system or receiving medical images that have been transmitted from a remote computer system.

At step 104 of Figure 1, a medical imaging analysis task is performed on the one or more input medical images using a trained AI system. The trained AI system is trained during a prior offline or training stage to perform a medical imaging analysis task on a set of training data in a central training database which may not reflect user-specific preferences. The set of training data is annotated by one or more users (e.g., radiologists). Once trained, the trained AI system is applied during an online or inference stage (e.g., at step 104 of Figure 1) to perform he medical imaging analysis task. In one example, as shown in workflow 200 of Figure 2, the trained AI system may be AI system 204 trained on database 202.

In one example, the medical imaging analysis task is the detection of abnormalities in chest x-ray input medical images. However, the medical imaging analysis task may comprise any other suitable medical imaging analysis task, such as, e.g., detection, classification, segmentation, quantification, etc.

At step 106 of Figure 1, feedback on results of the medical imaging analysis task is received from a user. In one example, the results of the medical imaging analysis task are displayed to the user on a display device and the feedback is received from the user interacting with the display device via a keyboard, mouse, touchscreen, etc. The feedback may comprise any suitable user feedback, such as, e.g., acceptance of the results, rejection of the results, editing of the results, and creation of new results (not generated by the trained AI system). The feedback is stored in a database associated with the user to thereby enable tracking of the feedback for the user. In one example, as shown in workflow 200 of Figure 2, the feedback may be feedback 206-A received from a first user and feedback 206-B received from a second user.

At step 108 of Figure 1, the trained AI system is retrained based on the feedback to generate a user-specific AI system for the user. In one embodiment, the trained AI system is retrained based on the feedback from only that single user (and not any other user). During the retraining, the feedback is used to fine-tune the trained AI system on the preferences of the user. In one example, as shown in Figure 1, the AI system 204 is retrained on feedback 206-A of the first user to generate user-specific AI system 208-A for the first user and on feedback 206-B of the second user to generate user-specific AI system 208-B for the second user.

At step 110 of Figure 1, the user-specific AI system is validated. The validation is performed to ensure that the user-specific AI system is not experiencing regression (e.g., suddenly missing detections of significant findings). The validation is performed by performing a medical imaging analysis validation task on a validation dataset using the user-specific AI system. The validation dataset includes ground truth annotations, which may be consensus findings by expert users (e.g., radiologists). The performance of the user-specific AI system is determined by comparing results of the medical imaging analysis validation task with the ground truth annotations. The validation is then performed by comparing the performance of the user-specific AI system with a performance threshold (e.g., 80 percent). The user-specific AI system is validated where the performance satisfies (e.g., is greater than or equal to) the performance threshold. If the user-specific AI system is not validated, the user-specific AI system is not deployed.

At step 112 of Figure 1, the validated user-specific AI system is output. In one embodiment, the validated user-specific AI system is output by deploying the validated user-specific AI system for use in a clinical setting. The validated user-specific AI system may then be applied to perform the medical imaging analysis task. However, the validated user-specific AI system may also be output by, for example, storing the validated user-specific AI system on a memory or storage of a computer system, or by transmitting the validated user-specific AI system to a remote computer system.

In one embodiment, the validated user-specific AI system may be periodically retrained based on feedback from the user by, for example, repeating the steps of method 100 of Figure 1 one or more iterations using the validated user-specific AI system as the trained AI system. The retraining may be performed according to a regular schedule, for example, at predetermined intervals of time or upon receiving a predetermined amount of feedback from the user.

Embodiments described herein are described with respect to the claimed systems as well as with respect to the claimed methods. Features, advantages or alternative embodiments herein can be assigned to the other claimed objects and vice versa. In other words, claims for the systems can be improved with features described or claimed in the context of the methods. In this case, the functional features of the method are embodied by objective units of the providing system.

Furthermore, certain embodiments described herein are described with respect to methods and systems utilizing trained AI systems (or machine learning based networks or models), as well as with respect to methods and systems for training AI systems. Features, advantages or alternative embodiments herein can be assigned to the other claimed objects and vice versa. In other words, claims for methods and systems for training an AI system can be improved with features described or claimed in context of the methods and systems for utilizing a trained AI system, and vice versa.

In particular, the trained AI system applied in embodiments described herein can be adapted by the methods and systems for training the AI systems. Furthermore, the input data of the trained AI systems can comprise advantageous features and embodiments of the training input data, and vice versa. Furthermore, the output data of the trained AI system can comprise advantageous features and embodiments of the output training data, and vice versa.

In general, a trained AI system mimics cognitive functions that humans associate with other human minds. In particular, by training based on training data, the trained AI system is able to adapt to new circumstances and to detect and extrapolate patterns.

In general, parameters of an AI system can be adapted by means of training. In particular, supervised training, semi-supervised training, unsupervised training, reinforcement learning and/or active learning can be used. Furthermore, representation learning (an alternative term is "feature learning") can be used. In particular, the parameters of the trained AI system can be adapted iteratively by several steps of training.

In particular, a trained AI system can comprise a neural network, a support vector machine, a decision tree, and/or a Bayesian network, and/or the trained machine learning based network can be based on k-means clustering, Q-learning, genetic algorithms, and/or association rules. In particular, a neural network can be a deep neural network, a convolutional neural network, or a convolutional deep neural network. Furthermore, a neural network can be an adversarial network, a deep adversarial network and/or a generative adversarial network.

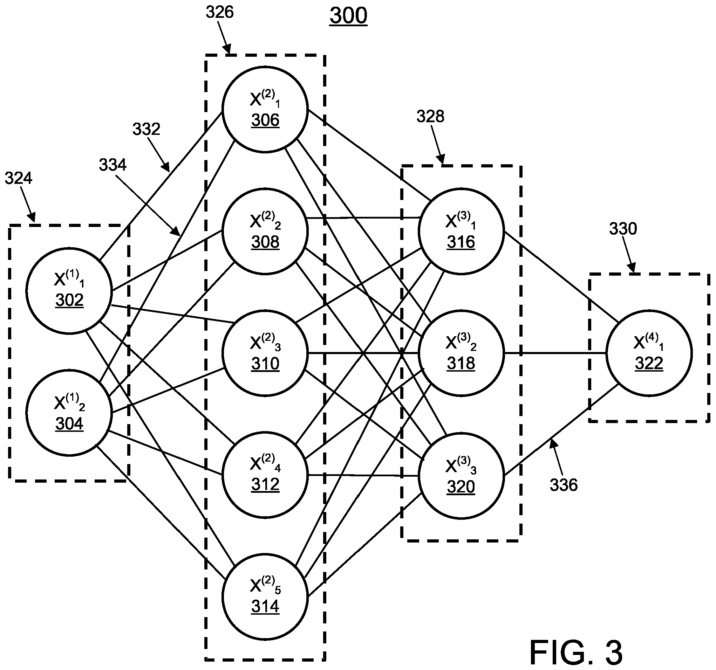

Figure 3 shows an embodiment of an artificial neural network 300, in accordance with one or more embodiments. Alternative terms for "artificial neural network" are "neural network", "artificial neural net" or "neural net". AI systems described herein, such as, e.g., the AI system in method 100 of Figure 1 and AI system 204 of workflow 200 of Figure 2, may be implemented using artificial neural network 300.

The artificial neural network 300 comprises nodes 302-322 and edges 332, 334, ..., 336, wherein each edge 332, 334, ..., 336 is a directed connection from a first node 302-322 to a second node 302-322. In general, the first node 302-322 and the second node 302-322 are different nodes 302-322, it is also possible that the first node 302-322 and the second node 302-322 are identical. For example, in Figure 3, the edge 332 is a directed connection from the node 302 to the node 306, and the edge 334 is a directed connection from the node 304 to the node 306. An edge 332, 334, ..., 336 from a first node 302-322 to a second node 302-322 is also denoted as "ingoing edge" for the second node 302-322 and as "outgoing edge" for the first node 302-322.

In this embodiment, the nodes 302-322 of the artificial neural network 300 can be arranged in layers 324-330, wherein the layers can comprise an intrinsic order introduced by the edges 332, 334, ..., 336 between the nodes 302-322. In particular, edges 332, 334, ..., 336 can exist only between neighboring layers of nodes. In the embodiment shown in Figure 3, there is an input layer 324 comprising only nodes 302 and 304 without an incoming edge, an output layer 330 comprising only node 322 without outgoing edges, and hidden layers 326, 328 in-between the input layer 324 and the output layer 330. In general, the number of hidden layers 326, 328 can be chosen arbitrarily. The number of nodes 302 and 304 within the input layer 324 usually relates to the number of input values of the neural network 300, and the number of nodes 322 within the output layer 330 usually relates to the number of output values of the neural network 300.

In particular, a (real) number can be assigned as a value to every node 302-322 of the neural network 300. Here, $x^{(n)}_i$ denotes the value of the i-th node 302-322 of the n-th layer 324-330. The values of the nodes 302-322 of the input layer 324 are equivalent to the input values of the neural network 300, the value of the node 322 of the output layer 330 is equivalent to the output value of the neural network 300. Furthermore, each edge 332, 334, ..., 336 can comprise a weight being a real number, in particular, the weight is a real number within the interval [-1, 1] or within the interval [0, 1]. Here, $w^{(m,n)}_{i,j}$ denotes the weight of the edge between the i-th node 302-322 of the m-th layer 324-330 and the j-th node 302-322 of the n-th layer 324-330. Furthermore, the abbreviation $w^{(n)}_{i,j}$ is defined for the weight $w^{(n,n+1)}_{i,j}$.

In particular, to calculate the output values of the neural network 300, the input values are propagated through the neural network. In particular, the values of the nodes 302-322 of the (n+1)-th layer 324-330 can be calculated based on the values of the nodes 302-322 of the n-th layer 324-330 by

$$x^{(n+1)}_j = f\left(\sum_i x^{(n)}_i \cdot w^{(n)}_{i,j}\right).$$

Herein, the function f is a transfer function (another term is "activation function"). Known transfer functions are step functions, sigmoid function (e.g. the logistic function, the generalized logistic function, the hyperbolic tangent, the Arctangent function, the error function, the smoothstep function) or rectifier functions. The transfer function is mainly used for normalization purposes.

In particular, the values are propagated layer-wise through the neural network, wherein values of the input layer 324 are given by the input of the neural network 300, wherein values of the first hidden layer 326 can be calculated based on the values of the input layer 324 of the neural network, wherein values of the second hidden layer 328 can be calculated based in the values of the first hidden layer 326, etc.

In order to set the values $w^{(m,n)}_{i,j}$ for the edges, the neural network 300 has to be trained using training data. In particular, training data comprises training input data and training output data (denoted as $t_i$). For a training step, the neural network 300 is applied to the training input data to generate calculated output data. In particular, the training data and the calculated output data comprise a number of values, said number being equal with the number of nodes of the output layer.

In particular, a comparison between the calculated output data and the training data is used to recursively adapt the weights within the neural network 300 (backpropagation algorithm). In particular, the weights are changed according to

$$w'^{(n)}_{i,j} = w^{(n)}_{i,j} - \gamma \cdot \delta^{(n)}_j \cdot x^{(n)}_i$$

wherein $\gamma$ is a learning rate, and the numbers $\delta^{(n)}_j$ can be recursively calculated as

$$\delta^{(n)}_j = \left(\sum_k \delta^{(n+1)}_k \cdot w^{(n+1)}_{j,k}\right) \cdot f\left(\sum_i x^{(n)}_i \cdot w^{(n)}_{i,j}\right)$$

based on $\delta^{(n+1)}_j$, if the (n+1)-th layer is not the output layer, and

$$\delta^{(n)}_j = \left(x^{(n+1)}_k - t^{(n+1)}_j\right) \cdot f\left(\sum_i x^{(n)}_i \cdot w^{(n)}_{i,j}\right)$$

if the (n+1)-th layer is the output layer 330, wherein f is the first derivative of the activation function, and $y^{(n+1)}_j$ is the comparison training value for the j-th node of the output layer 330.

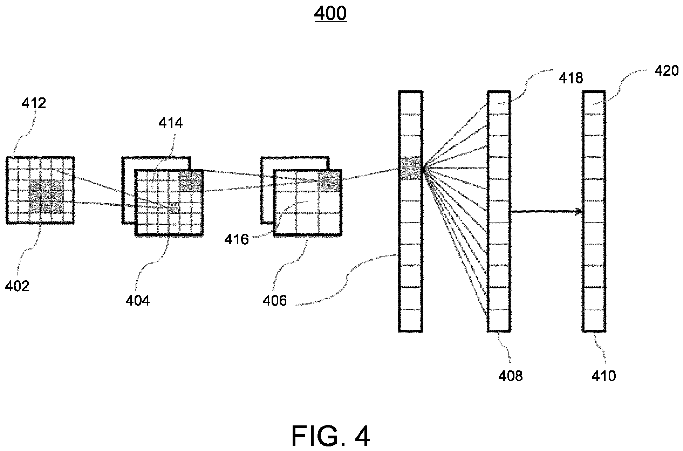

Figure 4 shows a convolutional neural network 400, in accordance with one or more embodiments. AI systems described herein, such as, e.g., the AI system in method 100 of Figure 1 and AI system 204 of workflow 200 of Figure 2, may be implemented using convolutional neural network 400.

In the embodiment shown in Figure 4, the convolutional neural network comprises 400 an input layer 402, a convolutional layer 404, a pooling layer 406, a fully connected layer 408, and an output layer 410. Alternatively, the convolutional neural network 400 can comprise several convolutional layers 404, several pooling layers 406, and several fully connected layers 408, as well as other types of layers. The order of the layers can be chosen arbitrarily, usually fully connected layers 408 are used as the last layers before the output layer 410.

In particular, within a convolutional neural network 400, the nodes 412-420 of one layer 402-410 can be considered to be arranged as a d-dimensional matrix or as a d-dimensional image. In particular, in the two-dimensional case the value of the node 412-420 indexed with i and j in the n-th layer 402-410 can be denoted as $x^{(n)}_{[i,j]}$. However, the arrangement of the nodes 412-420 of one layer 402-410 does not have an effect on the calculations executed within the convolutional neural network 400 as such, since these are given solely by the structure and the weights of the edges.

In particular, a convolutional layer 404 is characterized by the structure and the weights of the incoming edges forming a convolution operation based on a certain number of kernels. In particular, the structure and the weights of the incoming edges are chosen such that the values $x^{(n)}_k$ of the nodes 414 of the convolutional layer 404 are calculated as a convolution $x^{(n)}_k = K_k * x^{(n-1)}$ based on the values $x^{(n-1)}$ of the nodes 412 of the preceding layer 402, where the convolution * is defined in the two-dimensional case as

$$x^{(n)}_k[i,j] = \left(K_k * x^{(n-1)}\right)[i,j] = \sum_{i'}\sum_{j'} K_k[i',j'] \cdot x^{(n-1)}[i-i', j-j'].$$

Here the k-th kernel $K_k$ is a d-dimensional matrix (in this embodiment a two-dimensional matrix), which is usually small compared to the number of nodes 412-418 (e.g. a 3x3 matrix, or a 5x5 matrix). In particular, this implies that the weights of the incoming edges are not independent, but chosen such that they produce said convolution equation. In particular, for a kernel being a 3x3 matrix, there are only 9 independent weights (each entry of the kernel matrix corresponding to one independent weight), irrespectively of the number of nodes 412-420 in the respective layer 402-410. In particular, for a convolutional layer 404, the number of nodes 414 in the convolutional layer is equivalent to the number of nodes 412 in the preceding layer 402 multiplied with the number of kernels.

If the nodes 412 of the preceding layer 402 are arranged as a d-dimensional matrix, using a plurality of kernels can be interpreted as adding a further dimension (denoted as "depth" dimension), so that the nodes 414 of the convolutional layer 404 are arranged as a (d+1)-dimensional matrix. If the nodes 412 of the preceding layer 402 are already arranged as a (d+1)-dimensional matrix comprising a depth dimension, using a plurality of kernels can be interpreted as expanding along the depth dimension, so that the nodes 414 of the convolutional layer 404 are arranged also as a (d+1)-dimensional matrix, wherein the size of the (d+1)-dimensional matrix with respect to the depth dimension is by a factor of the number of kernels larger than in the preceding layer 402.

The advantage of using convolutional layers 404 is that spatially local correlation of the input data can exploited by enforcing a local connectivity pattern between nodes of adjacent layers, in particular by each node being connected to only a small region of the nodes of the preceding layer.

In embodiment shown in Figure 4, the input layer 402 comprises 36 nodes 412, arranged as a two-dimensional 6x6 matrix. The convolutional layer 404 comprises 72 nodes 414, arranged as two two-dimensional 6x6 matrices, each of the two matrices being the result of a convolution of the values of the input layer with a kernel. Equivalently, the nodes 414 of the convolutional layer 404 can be interpreted as arranges as a three-dimensional 6x6x2 matrix, wherein the last dimension is the depth dimension.

A pooling layer 406 can be characterized by the structure and the weights of the incoming edges and the activation function of its nodes 416 forming a pooling operation based on a non-linear pooling function f. For example, in the two dimensional case the values $x^{(n)}$ of the nodes 416 of the pooling layer 406 can be calculated based on the values $x^{(n-1)}$ of the nodes 414 of the preceding layer 404 as

$$x^{(n)}[i,j] = f(x^{(n-1)}[id_1, jd_2], \ldots, x^{(n-1)}[id_1+d_1-1, jd_2+d_2-1])$$

In other words, by using a pooling layer 406, the number of nodes 414, 416 can be reduced, by replacing a number d1·d2 of neighboring nodes 414 in the preceding layer 404 with a single node 416 being calculated as a function of the values of said number of neighboring nodes in the pooling layer. In particular, the pooling function f can be the max-function, the average or the L2-Norm. In particular, for a pooling layer 406 the weights of the incoming edges are fixed and are not modified by training.

The advantage of using a pooling layer 406 is that the number of nodes 414, 416 and the number of parameters is reduced. This leads to the amount of computation in the network being reduced and to a control of overfitting.

In the embodiment shown in Figure 4, the pooling layer 406 is a max-pooling, replacing four neighboring nodes with only one node, the value being the maximum of the values of the four neighboring nodes. The max-pooling is applied to each d-dimensional matrix of the previous layer; in this embodiment, the max-pooling is applied to each of the two two-dimensional matrices, reducing the number of nodes from 72 to 18.

A fully-connected layer 408 can be characterized by the fact that a majority, in particular, all edges between nodes 416 of the previous layer 406 and the nodes 418 of the fully-connected layer 408 are present, and wherein the weight of each of the edges can be adjusted individually.

In this embodiment, the nodes 416 of the preceding layer 406 of the fully-connected layer 408 are displayed both as two-dimensional matrices, and additionally as non-related nodes (indicated as a line of nodes, wherein the number of nodes was reduced for a better presentability). In this embodiment, the number of nodes 418 in the fully connected layer 408 is equal to the number of nodes 416 in the preceding layer 406. Alternatively, the number of nodes 416, 418 can differ.

Furthermore, in this embodiment, the values of the nodes 420 of the output layer 410 are determined by applying the Softmax function onto the values of the nodes 418 of the preceding layer 408. By applying the Softmax function, the sum the values of all nodes 420 of the output layer 410 is 1, and all values of all nodes 420 of the output layer are real numbers between 0 and 1.

A convolutional neural network 400 can also comprise a ReLU (rectified linear units) layer or activation layers with non-linear transfer functions. In particular, the number of nodes and the structure of the nodes contained in a ReLU layer is equivalent to the number of nodes and the structure of the nodes contained in the preceding layer. In particular, the value of each node in the ReLU layer is calculated by applying a rectifying function to the value of the corresponding node of the preceding layer.

The input and output of different convolutional neural network blocks can be wired using summation (residual / dense neural networks), element-wise multiplication (attention) or other differentiable operators. Therefore, the convolutional neural network architecture can be nested rather than being sequential if the whole pipeline is differentiable.

In particular, convolutional neural networks 400 can be trained based on the backpropagation algorithm. For preventing overfitting, methods of regularization can be used, e.g. dropout of nodes 412-420, stochastic pooling, use of artificial data, weight decay based on the L1 or the L2 norm, or max norm constraints. Different loss functions can be combined for training the same neural network to reflect the joint training objectives. A subset of the neural network parameters can be excluded from optimization to retain the weights pretrained on another datasets.

Systems, apparatuses, and methods described herein may be implemented using digital circuitry, or using one or more computers using well-known computer processors, memory units, storage devices, computer software, and other components. Typically, a computer includes a processor for executing instructions and one or more memories for storing instructions and data. A computer may also include, or be coupled to, one or more mass storage devices, such as one or more magnetic disks, internal hard disks and removable disks, magneto-optical disks, optical disks, etc.

Systems, apparatus, and methods described herein may be implemented using computers operating in a client-server relationship. Typically, in such a system, the client computers are located remotely from the server computer and interact via a network. The client-server relationship may be defined and controlled by computer programs running on the respective client and server computers.

Systems, apparatus, and methods described herein may be implemented within a network-based cloud computing system. In such a network-based cloud computing system, a server or another processor that is connected to a network communicates with one or more client computers via a network. A client computer may communicate with the server via a network browser application residing and operating on the client computer, for example. A client computer may store data on the server and access the data via the network. A client computer may transmit requests for data, or requests for online services, to the server via the network. The server may perform requested services and provide data to the client computer(s). The server may also transmit data adapted to cause a client computer to perform a specified function, e.g., to perform a calculation, to display specified data on a screen, etc. For example, the server may transmit a request adapted to cause a client computer to perform one or more of the steps or functions of the methods and workflows described herein, including one or more of the steps or functions of Figures 1 or 2. Certain steps or functions of the methods and workflows described herein, including one or more of the steps or functions of Figures 1 or 2, may be performed by a server or by another processor in a network-based cloud-computing system. Certain steps or functions of the methods and workflows described herein, including one or more of the steps of Figures 1 or 2, may be performed by a client computer in a network-based cloud computing system. The steps or functions of the methods and workflows described herein, including one or more of the steps of Figures 1 or 2, may be performed by a server and/or by a client computer in a network-based cloud computing system, in any combination.

Systems, apparatus, and methods described herein may be implemented using a computer program product tangibly embodied in an information carrier, e.g., in a non-transitory machine-readable storage device, for execution by a programmable processor; and the method and workflow steps described herein, including one or more of the steps or functions of Figures 1 or 2, may be implemented using one or more computer programs that are executable by such a processor. A computer program is a set of computer program instructions that can be used, directly or indirectly, in a computer to perform a certain activity or bring about a certain result. A computer program can be written in any form of programming language, including compiled or interpreted languages, and it can be deployed in any form, including as a stand-alone program or as a module, component, subroutine, or other unit suitable for use in a computing environment.

A high-level block diagram of an example computer 502 that may be used to implement systems, apparatus, and methods described herein is depicted in Figure 5. Computer 502 includes a processor 504 operatively coupled to a data storage device 512 and a memory 510. Processor 504 controls the overall operation of computer 502 by executing computer program instructions that define such operations. The computer program instructions may be stored in data storage device 512, or other computer readable medium, and loaded into memory 510 when execution of the computer program instructions is desired. Thus, the method and workflow steps or functions of Figures 1 or 2 can be defined by the computer program instructions stored in memory 510 and/or data storage device 512 and controlled by processor 504 executing the computer program instructions. For example, the computer program instructions can be implemented as computer executable code programmed by one skilled in the art to perform the method and workflow steps or functions of Figures 1 or 2. Accordingly, by executing the computer program instructions, the processor 504 executes the method and workflow steps or functions of Figures 1 or 2. Computer 502 may also include one or more network interfaces 506 for communicating with other devices via a network. Computer 502 may also include one or more input/output devices 508 that enable user interaction with computer 502 (e.g., display, keyboard, mouse, speakers, buttons, etc.).

Processor 504 may include both general and special purpose microprocessors, and may be the sole processor or one of multiple processors of computer 502. Processor 504 may include one or more central processing units (CPUs), for example. Processor 504, data storage device 512, and/or memory 510 may include, be supplemented by, or incorporated in, one or more application-specific integrated circuits (ASICs) and/or one or more field programmable gate arrays (FPGAs).

Data storage device 512 and memory 510 each include a tangible non-transitory computer readable storage medium. Data storage device 512, and memory 510, may each include high-speed random access memory, such as dynamic random access memory (DRAM), static random access memory (SRAM), double data rate synchronous dynamic random access memory (DDR RAM), or other random access solid state memory devices, and may include non-volatile memory, such as one or more magnetic disk storage devices such as internal hard disks and removable disks, magneto-optical disk storage devices, optical disk storage devices, flash memory devices, semiconductor memory devices, such as erasable programmable read-only memory (EPROM), electrically erasable programmable read-only memory (EEPROM), compact disc read-only memory (CD-ROM), digital versatile disc read-only memory (DVD-ROM) disks, or other non-volatile solid state storage devices.

Input/output devices 508 may include peripherals, such as a printer, scanner, display screen, etc. For example, input/output devices 508 may include a display device such as a cathode ray tube (CRT) or liquid crystal display (LCD) monitor for displaying information to the user, a keyboard, and a pointing device such as a mouse or a trackball by which the user can provide input to computer 502.

An image acquisition device 514 can be connected to the computer 502 to input image data (e.g., medical images) to the computer 502. It is possible to implement the image acquisition device 514 and the computer 502 as one device. It is also possible that the image acquisition device 514 and the computer 502 communicate wirelessly through a network. In a possible embodiment, the computer 502 can be located remotely with respect to the image acquisition device 514.

Any or all of the systems and apparatus discussed herein may be implemented using one or more computers such as computer 502.

One skilled in the art will recognize that an implementation of an actual computer or computer system may have other structures and may contain other components as well, and that Figure 5 is a high level representation of some of the components of such a computer for illustrative purposes.

Independent of the grammatical term usage, individuals with male, female or other gender identities are included within the term.

The foregoing Detailed Description is to be understood as being in every respect illustrative and exemplary, but not restrictive, and the scope of the invention disclosed herein is not to be determined from the Detailed Description, but rather from the claims as interpreted according to the full breadth permitted by the patent laws. It is to be understood that the embodiments shown and described herein are only illustrative of the principles of the present invention and that various modifications may be implemented by those skilled in the art without departing from the scope of the invention. Those skilled in the art could implement various other feature combinations without departing from the scope of the invention.

**Claims**

1.  A computer-implemented method comprising:

    receiving (102) one or more input medical images;
    performing (104) a medical imaging analysis task on the one or more input medical images using a trained AI (artificial intelligence) system (204);
    receiving (106) feedback (206-A, 206-B) on results of the medical imaging analysis task from a user;

retraining (108) the trained AI system based on the feedback to generate a user-specific AI system (208-A, 208-B) for the user;
validating (110) the user-specific AI system; and
outputting (112) the validated user-specific AI system.

2. The computer-implemented method of claim 1, wherein validating the user-specific AI system comprises:

performing a medical imaging analysis validation task on a validation dataset using the user-specific AI system;
determining a performance of the user-specific AI system by comparing results of the medical imaging analysis validation task with ground truth annotations of the validation dataset; and
comparing the performance of the user-specific AI system with a performance threshold.

3. The computer-implemented method of claim 1 or 2, wherein the feedback comprises one or more of acceptance of the results, rejection of the results, editing of the results, or creation of new results.

4. The computer-implemented method of any one of claims 1-3, wherein receiving feedback on results of the medical imaging analysis task from a user comprises:
storing the feedback on a database associated with the user.

5. The computer-implemented method of any one of claims 1-4, wherein retraining the trained AI system based on the feedback to generate a user-specific AI system for the user comprises:
retraining the trained AI system based on the feedback from the only the user.

6. The computer-implemented method of any one of claims 1-5, wherein outputting the validated user-specific AI system comprises:
deploying the validated user-specific AI system for use in a clinical setting.

7. The computer-implemented method of any one of claims 1-6, wherein the medical imaging analysis task comprises detection of abnormalities.

8. An apparatus comprising:

means for receiving (102) one or more input medical images;
means for performing (104) a medical imaging analysis task on the one or more input medical images using a trained AI (artificial intelligence) system (204);
means for receiving (106) feedback (206-A, 206-B) on results of the medical imaging analysis task from a user;
means for retraining (108) the trained AI system based on the feedback to generate a user-specific AI system (208-A, 208-B) for the user;
means for validating (110) the user-specific AI system; and means for outputting (112) the validated user-specific AI system.

9. The apparatus of claim 8, wherein the means for validating the user-specific AI system comprises:

means for performing a medical imaging analysis validation task on a validation dataset using the user-specific AI system;
means for determining a performance of the user-specific AI system by comparing results of the medical imaging analysis validation task with ground truth annotations of the validation dataset; and
means for comparing the performance of the user-specific AI system with a performance threshold.

10. The apparatus of claim 8 or 9, wherein the feedback comprises one or more of acceptance of the results, rejection of the results, editing of the results, or creation of new results.

11. The apparatus of any one of claims 8 - 10, wherein the means for receiving feedback on results of the medical imaging analysis task from a user comprises:
means for storing the feedback on a database associated with the user.

12. The apparatus of any one of claims 8 - 11, wherein the means for retraining the trained AI system based on the feedback to generate a user-specific AI system for the user comprises:

means for retraining the trained AI system based on the feedback from only the user.

13. The apparatus of any one of claims 8 - 12, wherein the means for outputting the validated user-specific AI system comprises:
means for deploying the validated user-specific AI system for use in a clinical setting.

14. The apparatus of any one of claims 8 - 13, wherein the medical imaging analysis task comprises detection of abnormalities.

15. A non-transitory computer readable medium storing computer program instructions, the computer program instructions when executed by a processor cause the processor to perform operations comprising the steps of the method according to any one of claims 1 - 7.

# FIG 1

100

```
Receive one or more input medical images
102
```

↓

```
Perform a medical imaging analysis task on
the one or more input medical images using a
trained AI system
104
```

↓

```
Receive feedback on results of the medical
imaging analysis task from a user
106
```

↓

```
Retrain the trained AI system based on the
feedback to generate a user-specific AI
system for the user
108
```

↓

```
Validate the user-specific AI system
110
```

↓

```
Output the user-specific AI system
112
```

FIG. 2

EP 4 404 207 A1

FIG. 3

400

FIG. 4

# FIG 5

502

Network
interface
506

I/O
508

Processor
504

Storage
512

Memory
510

Image Acquisition
Device
514

Europäisches
Patentamt

European
Patent Office

Office européen
des brevets

**EUROPEAN SEARCH REPORT**

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2015/031576 A1 (SIEMENS AG [DE]; SIEMENS CORP [US]) 5 March 2015 (2015-03-05) | 1-6, 8-13,15 | INV. G16H30/40 G06N3/045 |
| Y | * paragraph [0055] - paragraph [0079] * | 7,14 | G16H50/20 G16H50/70 |
| X | FLORIN CRISTIAN GHESU ET AL: "Multi-Scale Deep Reinforcement Learning for Real-Time 3D-Landmark Detection in CT Scans", IEEE TRANSACTIONS ON PATTERN ANALYSIS AND MACHINE INTELLIGENCE, 1 January 2017 (2017-01-01), pages 1-1, XP055484826, USA ISSN: 0162-8828, DOI: 10.1109/TPAMI.2017.2782687 | 1-6, 8-13,15 | |
| Y | * page 1 - page 9 * | 7,14 | |
| X | US 2022/028063 A1 (GUENDEL SEBASTIAN [DE] ET AL) 27 January 2022 (2022-01-27) | 1-6, 8-13,15 | |
| Y | * the whole document * | 7,14 | |

**TECHNICAL FIELDS SEARCHED (IPC)**

G16H
G06N

The present search report has been drawn up for all claims

| Place of search | Date of completion of the search | Examiner |
|---|---|---|
| Munich | 5 June 2024 | Eichenauer, Lars |

CATEGORY OF CITED DOCUMENTS

X : particularly relevant if taken alone
Y : particularly relevant if combined with another
   document of the same category
A : technological background
O : non-written disclosure
P : intermediate document

T : theory or principle underlying the invention
E : earlier patent document, but published on, or
   after the filing date
D : document cited in the application
L : document cited for other reasons
   ................................................................
& : member of the same patent family, corresponding
   document

EPO FORM 1503 03.82 (P04C01)

## ANNEX TO THE EUROPEAN SEARCH REPORT
## ON EUROPEAN PATENT APPLICATION NO.

EP 24 15 2572

This annex lists the patent family members relating to the patent documents cited in the above-mentioned European search report.
The members are as contained in the European Patent Office EDP file on
The European Patent Office is in no way liable for these particulars which are merely given for the purpose of information.

05-06-2024

| Patent document cited in search report | | Publication date | Patent family member(s) | | Publication date |
|---|---|---|---|---|---|
| WO 2015031576 | A1 | 05-03-2015 | CN | 105474219 A | 06-04-2016 |
| | | | KR | 20160060657 A | 30-05-2016 |
| | | | US | 2016210435 A1 | 21-07-2016 |
| | | | WO | 2015031576 A1 | 05-03-2015 |
| US 2022028063 | A1 | 27-01-2022 | CN | 113971657 A | 25-01-2022 |
| | | | EP | 3944253 A1 | 26-01-2022 |
| | | | US | 2022028063 A1 | 27-01-2022 |

EPO FORM P0459

For more details about this annex : see Official Journal of the European Patent Office, No. 12/82